(19) **Europäisches Patentamt**

**European Patent Office**

**Office européen des brevets**

(11) **EP 0 625 496 B1**

(12) **EUROPEAN PATENT SPECIFICATION**

(45) Date of publication and mention
of the grant of the patent:
**09.04.1997 Bulletin 1997/15**

(51) Int. Cl.$^6$: **C07C 17/361**, C07C 19/01,
C07C 68/00, C07C 67/38,
C07C 69/96, C07C 69/34,
C07C 69/40

(21) Application number: **94107893.3**

(22) Date of filing: **21.05.1994**

(54) **Method of decomposing an alkyl chloroformate**

Verfahren zur Zersetzung von Chlorameisensäurealkylester

Procédé de décomposition d'un chloroformiate d'alkyle

(84) Designated Contracting States:
**DE ES FR GB IT**

(30) Priority: **21.05.1993 JP 119870/93**
**21.05.1993 JP 119871/93**

(43) Date of publication of application:
**23.11.1994 Bulletin 1994/47**

(73) Proprietor: **UBE INDUSTRIES, LTD.**
**Ube-shi, Yamaguchi-ken 755 (JP)**

(72) Inventors:
• **Nishihira, Keigo,**
**c/o Ube Chemical Factory**
**Ube-shi, Yamaguchi (JP)**
• **Tanaka, Shuji,**
**c/o Ube Chemical Factory**
**Ube-shi, Yamaguchi (JP)**

• **Nishida, Yuki,**
**c/o Ube Chemical Factory**
**Ube-shi, Yamaguchi (JP)**
• **Manada, Noriaki,**
**c/o Ube Research Lab.**
**Ube-shi, Yamaguchi (JP)**
• **Kurafuji, Toshio,**
**c/o Ube Research Lab.**
**Ube-shi, Yamaguchi (JP)**
• **Murakami, Masato,**
**c/o Ube Research Lab.**
**Ube-shi, Yamaguchi (JP)**

(74) Representative: **Wössner, Gottfried**
**Hoeger, Stellrecht & Partner,**
**Uhlandstrasse 14c**
**70182 Stuttgart (DE)**

(56) References cited:
**EP-A- 0 425 197**     **BE-A- 651 529**
**GB-A- 2 024 821**     **US-A- 4 814 524**

## Description

### 1. Field of the Invention

The present invention relates to a method of decomposing a chloroformic acid ester.

More particularly, the present invention relates to a method of decomposing a chloroformic acid alkyl ester into a corresponding alkyl chloride, which method is useful to refine various mixed gases containing a chloroformic acid alkyl ester in various concentrations by bringing the chloroformic acid alkyl ester-containing gas into contact with a specific catalyst and thereby converting the chloroformic acid alkyl ester to an alkyl chloride and removing the alkyl chloride from the mixed gas.

The method of the present invention is applicable to a mixed gas produced from a gas phase catalytic carbonylation reaction of carbon monoxide with at least a nitrous acid ester in the presence of a solid catalyst and a chlorine compound, and containing, as a by-product, a chloroformic acid alkyl ester in addition to the resultant carbonylation product, for example, dialkyl carbonate or dialkyl succinate. The method of the present invention is contributory to preventing corrosion of a refining apparatus due to the chloroformic acid alkyl ester contained in the gas mixture.

### 2. Description of Related Art

As a known method of decomposing a chloroformic acid ester, J. Am. Chem. Soc., 77, 5033 (1955) discloses a method of converting a chloroformic acid ester to an alkyl chloride by heating the chloroformic acid ester in the presence of a Lewis acid such as boron trifluoride. This method is disadvantageous in that a long time is necessary for the decomposition of a lower alcohol ester of chloroformic acid and the resultant degree of decomposition is low.

Also, German Unexamined Patent Publication (DE-OS) No. 2,545,659 discloses a method of producing an alkyl chloride by heating a chloroformic acid ester at a temperature of 120 to 130°C in an aprotic solvent, for example, N-methylpyrrolidone. This method is disadvantageous in that this is a liquid phase method in which a special solvent must be employed and a solvent vessel must be provided, and thus is not economical for industrially decomposing the chloroformic acid ester.

U.S. Patent No. 4,260,810 and U.S. Patent No. 5,162,563 disclose that in catalytic carbonylationn reactions, for example, carbonic acid ester-synthesis reactions or dicarboxylic acid diester-synthesis reactions, of carbon monoxide with at least nitrous acid esters in a gas phase in the presence of a solid catalyst comprising a platinum group metal chloride carried on a carrier, the catalytic activity of the solid catalyst gradually decreases during the reaction, and in the industrial procedure, a small amount of a chlorine compound, for example, hydrogen chloride, chlorine or a chloroformic acid ester, is continuously fed into the reaction system to prolong the durability of the catalyst and to maintain its selectivity for the targeted compound at a desired level or to enhance its selectivity.

In the above-mentioned carbonylation reaction procedures, the resultant reaction product mixture delivered from the reaction system contains a small amount of a chloroformic acid alkyl ester or a small amount of a chloroformic acid alkyl ester added to the reaction system, in addition to the aimed reaction product, for example, a carbonic acid ester or dicarboxylic acid diester, non-reacted fractions of carbon monoxide and nitrous acid ester, and another additive, for example, nitrogen monoxide and nitrogen, and therefore, the chloroformic acid alkyl ester is brought together with the targeted ester compound into a collecting and refining process for the targeted ester compound. Where the chloroformic acid alkyl ester, which has a poor resistance to hydrolysis or alcoholysis, is introduced into the collecting and refining process, the chloroformic acid alkyl ester is decomposed to a corresponding chlorine compound, for example, hydrogen chloride, and the chlorine compound contaminates the targeted product and corrodes the apparatus. Therefore, in this case, it is required to further refine the product to remove the chlorine compound, or to make the refining process apparatus by a specific material having a high resistance to the chlorine compound. Namely, the chlorine compound contained in an industrial product causes significant disadvantages in cost and/or production efficiency.

Nevertheless, with respect to the carbonylation reaction product containing a small amount of a chloroformic acid alkyl ester, it has, until now, not been known how to decompose it to a substantially non-corrosive substance with a high economical efficiency.

Accordingly, there is a strong demand for a names method of decomposing chloroformic acid alkyl esters to the substantially non-corrosive substance efficiently and economically, which method is useful, for example, for refining a reaction product mixture derived from carbonylation reactions of carbon monoxide with at least a nitrous acid ester.

### SUMMARY OF THE INVENTION

An object of the present invention is to provide a method of decomposing a chloroformic acid alkyl ester to a corresponding alkyl cloride at high efficiency and low cost.

Another object of the present invention is to provide a method of decomposing a chloroformic acid alkyl ester contained in a reaction product mixture derived from a carbonylation reaction of carbon monoxide with at least a nitrous acid ester, the decomposition product consisting of a corresponding alkyl chloride being removed from the carbonylation reaction product mixture efficiently and economically.

The above-mentioned objects can be attained by the method of the present invention of decomposing a chloroformic acid alkyl ester, which method comprises the step of bringing a gas containing a chloroformic acid alkyl ester into contact with a decomposition catalyst comprising at least one member selected from the group consisting of activated carbon and inorganic oxides, to convert the chloroformic acid alkyl ester to a corresponding alkyl chloride.

The method of the present invention is useful for decomposing a chloroformic acid alkyl ester contained in a reaction product mixture gas derived from a gas phase catalytic carbonylation reaction of carbon monoxide with a nitrous acid ester in the presence of a solid catalyst and in the presence of a small amount of a chlorine-containing substance, the resultant alkyl chloride being removed from the carbonylation reaction product mixture.

The gas phase catalytic carbonylation reaction may be a carbonic acid ester-synthesis reaction, a dicarboxylic acid diester-synthesis reaction or a succinic acid diester synthesis reaction.

Usually, the chlorine-containing substance comprises at least one member selected from the group consisting of chlorine, hydrogen chloride and chloroformic acid alkyl esters.

Preferably, the chloroformic acid alkyl ester is a reaction product mixture derived from a gas phase carbonic acid ester-synthesis reaction and the chlorine-containing substance is hydrogen chloride.

## DESCRIPTION OF THE PREFERRED EMBODIMENTS

In the method of the present invention, a gas containing a chloroformic acid alkyl ester is brought into contact with a solid catalyst consisting of at least one member selected from activated carbon and inorganic oxides, to convert the chloroformic acid ester to a corresponding alkyl chloride.

The chloroformic acid alkyl ester to which the method of the present invention is applicable is usually a lower alkyl chloroformate of which the alkyl group preferably has 1 to 4 carbon atoms. The lower alkyl chloroformate is selected from methyl chloroformate, ethyl chloroformate, n and iso-propyl chloroformates, n-, iso-, sec- and tert-butyl chloroformates. Namely, the alkyl group of the lower alkyl chloroformate is preferably selected from methyl, ethyl, n-propyl, iso-propyl, n-butyl, iso-butyl, sec-butyl and tert-butyl groups.

In the chloroformic acid alkyl ester-containing gas, to which the method of the present invention is applicable, the concentration of the chloroformic acid alkyl ester is not limited to a specific range thereof. For example, the chloroformic acid alkyl ester concentration is variable through a wide range of from 10 ppm to 100% by volume, preferably from 20 ppm to 50% by volume.

Especially, the decomposition method of the present invention is advantageously applicable to a gaseous mixture containing the chloroformic acid alkyl ester in a low concentration of from 20 to 10,000 ppm, preferably 50 to 5,000 ppm.

In the method of the present invention, the gas substance which can be contained together with the chloroformic acid alkyl ester in the mixed gas to which the method of the present invention is applied, is not restricted to a specific group of substance. For example, the gas substance which can be mixed with the chloroformic acid alkyl ester is an inert gas, for example, nitrogen, or carbon dioxide gas, and/or a reactive gas, for example, carbon monoxide, nitrous acid ester, alcohol or oxygen gas.

The alkyl chloride produced from the decomposition of the chloroformic acid alkyl ester is variable depending on the type of the chloroformic acid alkyl ester, and is preferably an alkyl chloride having 1 to 4 carbon atoms, for example, methyl chloride, ethyl chloride, n- or iso-propyl chloride or n-, iso-, sec- or tert-butyl chloride.

In the decomposition method of the present invention, the catalyst comprises at least one member selected from activated carbon and inorganic oxides. The catalyst preferably consists of an activated carbon. There is no limitation to the type, form, size, physical properties, for example, specific surface area and density, and/or chemical properties, for example, acid strength, of the activated carbon, as long as the activated carbon is selected from those usable as a catalyst carrier, an absorbent or decoloring agent. Preferably, the activated carbon used as a catalyst for the method of the present invention has a specific surface area of 10 $m^2$/g or more, more preferably 100 to 3,000 $m^2$/g, still more preferably 300 to 2,000 $m^2$/g and is in the form of grains, pellets or honeycombs.

The inorganic oxides usable as a catalyst for the method of the present invention are not restricted to those having specific form, size, physical properties, for example, specific surface area and density and/or chemical properties, for examples, acid strength. Preferably, the inorganic oxides are in the form of grains, pellets or honeycombs and have a specific surface area of 10 $m^2$/g or more, more preferably 100 to 1,000 $m^2$/g, still more preferably 300 to 800 $m^2$/g.

The inorganic oxides are preferably selected from those usable as a catalyst carrier, for example, various types of aluminas including $\gamma$-alumina, $\alpha$-alumina, and $\eta$-alumina, silica-alumina, synthetic zeolites, for example, X, Y and A-type synthetic zeolites and molecular sieves, natural zeolites, for example, natrolite and mordenite, silica, titania and zirconia.

The decomposition catalyst preferably comprises at least one member selected from activated carbon, and aluminum-containing inorganic oxides, for example, $\gamma$-alumina, $\alpha$-alumina, $\eta$-alumina and zeolites, in the form of grains.

The decomposition reaction of the chloroformic acid alkyl ester into a corresponding alkyl chloride, in accordance with the present invention can be carried out in a batch type procedure. From an industrial point of view, the decomposition reaction is preferably carried out by a gas phase continuous procedure. In this continuous procedure, the catalyst can be employed in a fixed bed or a fluidized bed.

In the method of the present invention, the decomposition step of the chloroformic acid alkyl ester into a corresponding alkyl chloride is carried out preferably at a gas hourly space velocity (GHSV) of the chlorofomic acid ester-containing gas of 1,000 to 30,000 $hr^{-1}$, more preferably 2,000 to 20,000 $hr^{-1}$, at a decomposition temperature of 0°C to 220°C, more preferably, 10°C to 200°C. Still more preferably, the decomposition temperature is maintained at a level ranging from 20°C to 150°C.

Since the dependency of the decomposition reaction on the pressure is low, the decomposition step can be carried out under a pressure of from the ambient atmospheric pressure to an increased pressure for example, from 0.98 to 19.61 bar (1 to 20 kg/cm$^2$G), preferably 1.96 to 4.90 bar (2 to 5 kg/cm$^2$G), without difficulty.

Where the decomposition reaction does not cause a generation of a large amount of heat, the contact of the chloroformic acid alkyl ester-containing gas with the catalyst can be effected in a multiple tube type reactor or a single tube type reactor in which the catalyst comprising the activated carbon and/or the inorganic oxide is filled. The decomposition reaction may be carried out in another type of reactor.

During the decomposition reaction step; substantially all of the chloroformic acid alkyl ester is converted to a corresponding alkyl chloride. Therefore, when the resultant alkyl chloride-containing gas is fed to a next step, corrosion of conduits and/or apparatus of the next step due to this gas is prevented or significantly reduced.

The method of the present invention is advantageously applied to a reaction product mixture gas derived from a gas phase catalytic carbonylation reaction of carbon monoxide with a nitrous acid ester in the presence of a solid carbonylation catalyst and in the presence of a small amount of a chlorine-containing substance, for example, hydrogen chloride, chlorine or chloroformic acid ester. The gas phase catalytic carbonylation reaction may be a carbonic acid ester synthesis reaction or a dicarboxylic acid diester synthesis reaction disclosed, for example, in U.S. Patent Nos. 4,260,810, and 5,162,563. The carbonylation reaction product mixture contains a small amount of a chloroformic acid alkyl ester, for example, lower alkyl chloroformate having 1 to 4 carbon atoms, methyl chloroformate, ethyl chloroformate, n- or iso-propyl chloroformate or n-, iso-, sec-, or tert-butyl chloroformate. The lower alkyl chloroformates are esters of monohydric lower alkyl alcohols having 1 to 4 carbon atoms with chloroformic acid. By the method of the present invention, the lower alkyl chloroformate in the reaction product mixture gas is converted to a corresponding lower alkyl chloride, for example, methyl chloride, ethyl chloride, n- or iso-propyl chloride or n-, iso-, or sec-, butyl chloride.

Where the carbonylation reaction product mixture gas is subjected to the decomposition method of the present invention, the decomposing catalyst may contain a metal component comprising a platinum group metal which is useful as a catalytically active component for the carbonylation catalyst, in a small amount in which the metal component cannot serve as a catalytic component. Preferably, the amount of the platinum group metal carried on the decomposition catalyst is restricted to a level of less than 0.1% by weight, more preferably 0 to 0.05% by weight based on the weight of the decomposition catalyst. Still more preferably, the platinum group metal is substantially not present in the decomposition catalyst.

The amount of the decomposition catalyst employed in a decomposition step is preferably 2 to 500%, more preferably 5 to 200%, still more preferably 10 to 50%, based on the volume of the carbonylation catalyst used for the carbonylation reaction.

The decomposition catalyst may be placed in the carbonylation reactor at a location close to an outlet of the carbonylation reactor. In this case, the carbonylation reaction and the decomposition reaction can be carried out in one and the same reactor and thus the reaction apparatus is very simple. Also, the decomposition catalyst may be placed in a decomposition reactor connected to the outlet of the carbonylation reactor in which the carbonylation catalyst is placed. For example, as disclosed in U.S. Patent No. 5,214,185, the decomposition reactor containing the decomposition catalyst is located in a conduit line between the carbonylation reactor for producing dimethyl carbonate and an absorption column.

In this case, since the amount of heat generated by the decomposition reaction is small, the decomposition reaction can be effected in a multiple tube type reactor, a single tube type reactor or another type of reactor without difficulty.

The feed gas for the decomposition step is preferably the reaction product mixture gas per se delivered from the carbonylation reaction system and containing a chloroformic acid alkyl ester produced from hydrogen chloride or chlorine added to the carbonylation reaction system. Accordingly, she reaction product mixture to be subjected to the decomposition step of the chloroformic acid alkyl ester contains a targeted carbonylation reaction product, for example, a carbonic acid ester or dicarboxylic acid diester, by-products, for example, oxalic acid diester, and others, for example, carbon monoxide, nitrous acid ester, nitrogen monoxide, carbon dioxide and nitrogen. These substances other than the chloroformic acid alkyl ester contained in the feed gas do not cause any problems for the decomposition reaction of the chloroformic acid ester.

The chlorine or chlorine compound added to the carbonylation reaction system serves as an activating agent for the solid carbonylation catalyst so as to maintain the catalytic activity of the catalyst at a desired high level in the carbonylation step, and then is converted to a chloroformic acid alkyl ester. By utilizing the decomposition method of the present invention, the chloroformic acid alkyl ester is converted to an alkyl chloride which is not corrosive to the apparatus and can be easily removed from the reaction product mixture.

For example, in a synthesis of dimethyl carbonate as disclosed in U.S. Patent No. 5,214,185, a decomposition device is located in a portion of a carbonylation reactor or in a portion of a conduit between the carbonylation reactor and an adsorption column, a carbonylation reaction product-containing gas is introduced into the decomposition device to convert methyl chloroformate contained in a small amount in the carbonylation reaction product-containing gas to methyl chloride, and the carbonylation reaction product containing methyl chloride is absorbed and the condensed by the absorption column.

The methyl chloride-containing carbonylation reaction product absorbed and condensed in the absorption column is fed to a distill-refining device connected to a bottom outlet of the absorption column. The distill-refining device is, for example, a distillation column or a multistep distillation column. The methyl chloride is discharged from a top outlet of the distill-refining device, and a crude product containing dimethyl carbonate is discharged from a bottom outlet thereof. The crude product has a significantly reduced content of methyl chloride and is subjected to a further refining step.

By the decomposition step for the chloroformic acid alkyl ester, the carbonylation reaction product, namely the targeted carbonic acid diester or dicarboxylic acid diester, is not influenced or converted, and can be supplied to the next step. Also, the decomposition step of the present invention can reduce the content of a chlorine compound, for example, hydrogen chloride, and thus is contributory to preventing an undesirable corrosion of apparatus due to the hydrogen chloride or other chlorine compound.

The catalytic carbonylation reaction for producing an ester compound from carbon monoxide and a nitrous acid ester can be easily carried out in gas phase by the method as disclosed in, for example, U.S. Patent No. 4,260,810, and U.S. Patent No. 5,162,563. The products of the carbonylation reaction include carbonic acid esters, for example, dimethyl carbonate and diethyl carbonate; and dicarboxylic acid diesters, for example, dimethyl succinate and diethyl succinate.

The carbonic acid diesters can be produced from carbon monoxide and a nitrous acid ester by the following catalytic carbonylation method.

The nitrous acid esters usable for the carbonic acid diester production include esters of nitrous acid with lower monohydric aliphatic alcohols having 1 to 4 carbon atoms, particularly, for example, methyl nitrite, ethyl nitrite, n-, or iso-propyl nitrite and n-, iso-, sec- or tert-butyl nitrite. In this carbonylation method, a chloroformic acid alkyl ester produced as a by-product and corresponding to the nitrous acid ester employed is contained in the resultant reaction product mixture.

The carbonylation reaction for producing the carbonic acid diester is carried out in the presence of a solid catalyst. This solid catalyst preferably comprises a catalytic metal component comprising at least one member selected from chlorides of platinum group metals, for example, palladium, platinum, iridium, ruthenium and rhodium, and optionally at least one member selected from compounds of iron, copper, bismuth, cobalt, nickel and tin, and a carrier comprising at least one member selected from activated carbon, alumina, diatomaceous earth, silicon carbide and titanium, the catalytic metal component being carried on the carrier.

The chlorides of the platinum group metals include particularly palladium chloride, platinum chloride, iridium chloride, ruthenium chloride and rhodium chloride. Among them, the palladium chloride, ruthenium chloride and rhodium chloride are preferably used and the palladium chloride is most preferably used for the carbonylation reaction. The amount of the platinum group metal chloride carried on the carrier is preferably 0.1 to 10% by weight, more preferably 0.5 to 2% by weight, based on the weight of the carrier.

In the catalytic component for the carbonylation reaction, the platinum group metal compounds are not limited to the above-mentioned chlorides, and can be selected from platinum group metals and compounds thereof which are capable of being converted, in the presence of hydrogen chloride, to the above-mentioned chlorides or chloride complexes capable of releasing therefrom chlorine which contributes to the carbonylation reaction.

The platinum group metal compounds include halides, for example, bromides, iodides and fluorides, inorganic acid salts, for example, nitrates, sulfates and phosphates, and organic acid salts, for example, acetates, oxalates and benzoates, of the platinum group metals. Particularly, the platinum group metal compounds are preferably selected from palladium bromide, palladium iodides, palladium fluorides, palladium nitrate, palladium sulfate, palladium phosphates, palladium oxalate and palladium benzoate.

The compounds of the metal group consisting of iron, copper, bismuth, cobalt, nickel and tin, usable for the carbonylation catalyst include halides, for example, chlorides, bromides, iodides and fluorides, inorganic acid salts, for example, nitrates, sulfates and phosphates, and organic acid salts, for example, acetates, of the above-mentioned metals. Preferably, the halides of the above-mentioned metals are employed. The metal compounds are contained preferably in an amount, in terms of the metal, of 1 to 50 gram atom equivalent, more preferably 1 to 10 gram atom equivalent, per gram atom equivalent of the platinum group metal.

When the carbonylation reaction system contains a small amount of hydrogen chloride, as a chlorine-containing substance, preferably the hydrogen chloride is anhydrous and added in an amount of 1 to 50 molar%, more preferably 5 to 20 molar% based on the molar amount of the platinum group metal contained in the solid catalyst, per unit time. For example, when the carbonylation reaction is carried out in a fixed bed reactor at a gas hour space velocity (GHSV) of 3000 hr$^{-1}$, preferably the feed material gas containing 10 to 500 ppm by volume, more preferably 20 to 100 ppm by volume of hydrogen chloride is continuously fed to the solid catalyst-placed reactor. Also, as a chlorine-containing substance, chlorine or a chloroformic acid ester is fed to the carbonylation reaction system to produce the carbonic acid diester.

In the feed material gas for the carbonylation reaction, preferably carbon monoxide and the nitrous acid ester are diluted with an inert gas and then supplied to the reactor. There is no limitation to the composition of the feed material gas. Usually, for safety, the concentration of the nitrous acid ester is restricted to 20% by volume or less, more preferably 5 to 20% by volume, and for economy, the concentration of carbon monoxide is limited to 5 to 20% by volume. The molar ratio of carbon monoxide to the nitrous acid ester in the feed material gas is preferably 0.1:1 to 10:1, more preferably 0.25:1 to 1:1. Additionally, the material gas is preferably fed into the reactor at a gas hourly space velocity (GHSV) of 500 to 20,000 hr$^{-1}$, more preferably 2,000 to 10,000 hr$^{-1}$.

The carbonylation reaction for producing an ester compound, for example, dimethyl carbonate is usually carried out under mild conditions, for example, at a temperature of from 0°C to 200°C, more preferably from 50°C to 120°C under ambient atmospheric pressure. Of course, the carbonylation reaction can be carried out under an increased pressure, for example, 1 to 20 kg/cm$^2$G, at a temperature of 50°C to 150°C.

The carbonylation reaction is preferably carried out continuously in a gas phase. The solid catalyst in the reactor may be in the state of a solid bed or a fluidized bed, and preferably in the form of grains having a mesh size of 4 to 200 or fine particles having a particle size of 20 to 100 μm.

U.S. Patent No. 5,162,563 discloses an embodiment of the synthesis of dimethyl carbonate. In this synthesis, solid catalyst grains comprising a carrier consisting of activated carbon grains and a catalytic metal component consisting of palladium chloride and bismuth chloride are placed in a gas phase reactor tube, and a feed material gas comprising 15% by volume of methyl nitrite, 10% by volume of carbon monoxide, 3% by volume of nitrogen monoxide, 6% by volume of methyl alcohol, 100 ppm by volume of hydrogen chloride and about 66% by volume of nitrogen, is fed into the gas phase reactor at a temperature of 100°C under the ambient atmospheric pressure at a gas hourly space velocity (GHSV) of 3000 hr$^{-1}$.

A process for producing a dicarboxylic acid diester, for example, dimethyl succinate is disclosed in, for example, U.S. Patent No. 5,162,563. In this process, a gas phase reactor tube is packed with solid catalyst consisting of silica beads (carrier) and metallic palladium and copper (II) chloride carried on the beads, and a mixed gas comprising 20% by volume of ethylene, 22% by volume of carbon monoxide, 5% by volume of nitrogen dioxide, and 30% by volume of oxygen is fed to the gas phase reactor tube at a temperature of 145°C under the ambient atmospheric pressure, at a feeding rate of 700 mℓ/min, and separately methyl alcohol and 36% by weight hydrochloric acid are vaporized and fed into the reactor at feed rates of 40 mℓ/min and 0.5 to 2 mℓ/min, respectively. The gas hourly space velocity (GHSV) of all the material gas is 2500 hr$^{-1}$.

In the catalytic carbonylation reaction of carbon monoxide with the nitrous acid ester in the presence of a solid catalyst, and in the additional presence of a small amount of a chlorine-containing substance such as chlorine, hydrogen chloride or a chloroformic acid ester, for the production of carbonic acid ester, for example, dimethyl carbonate or dicarboxylic acid ester, for example, dimethyl succinate, the resultant carbonylation product mixture gas contains, in addition to the targeted ester compound, non-reacted carbon monoxide and nitrous acid ester, and others, for example, nitrogen monoxide, carbon dioxide, an inert gas and a chloroformic acid ester. This carbonylation product mixture gas is fed to the decomposition step of the present invention in which the gas is brought into contact with the decomposition solid catalyst. In this decomposition step, the chloroformic acid ester is selectively decomposed, without affecting the targeted carbonylation product. This decomposition step effectively contributes to reducing the content of hydrogen chloride in the carbonylation product mixture gas.

The resultant carbonylation product mixture gas delivered from the decomposition step is cool-condensed. The targeted carbonylation product is collected from the condensed liquid by a collecting means, for example, distillation. The obtained product has a very small content of the chlorine-containing substance.

## EXAMPLES

The present invention will be further explained by the following examples which are merely representative of the present invention.

## Example 1

A gas phase glass tube reactor having an inside diameter of 13 mm and a length of 250 mm and equipped with an outside heating jacket was packed with 5 mℓ of a granular activated carbon having a specific surface area of 1040 m$^2$/g. The inside temperature of the reactor tube was controlled to a level of 120°C by circulating a heating medium through the outside heating jacket. A mixed gas containing 500 ppm by volume of methyl chloroformate diluted by a nitrogen gas was fed into the reactor tube through a top inlet thereof at a gas hourly space velocity (GHSV) of 4000 $hr^{-1}$, and brought into contact with the decomposition catalyst at a temperature of 120°C under the ambient atmospheric pressure for one hour, to decompose the methyl chloroformate.

The resultant mixed gas was delivered from the reactor and subjected to a gas chromatographic analysis. It was confirmed that the resultant mixed gas contained no methyl chloroformate and 500 ppm by volume of methyl chloride. Namely, 100% of the methyl chloroformate was decomposed and converted to methyl chloride.

Example 2

The same procedures as in Example 1 were carried out with the following exceptions.

The granular activated carbon was replaced by 1 m$\ell$ of granular activated alumina having a 24 mesh size.

The concentration of methyl chloroformate was changed to 840 ppm by volume.

The gas hourly space velocity (GHSV) of the mixed gas was changed to 20,000 $hr^{-1}$.

In the resultant mixed gas, no methyl chloroformate was detected, and the concentration of methyl chloride was 840 ppm by volume. It was confirmed that the methyl chloroformate was entirely decomposed.

Example 3

The same procedures as in Example 1 were carried out with the following exceptions.

The granular activated carbon was replaced by 1 m$\ell$ of granular activated alumina having a 24 mesh size.

The concentration of methyl chloroformate was changed to 800 ppm by volume.

The gas hourly space velocity (GHSV) of the mixed gas was changed to 20,000 $hr^{-1}$. The reaction temperature was changed to 80°C.

In the resultant mixed gas, the concentration of methyl chloroformate was 260 ppm by volume, and the concentration of methyl chloride was 540 ppm by volume. Namely, methyl chloroformate was decomposed at a conversion of 65%.

Example 4

The same procedures as in Example 1 were carried out with the following exceptions.

The granular activated carbon was replaced by 5 m$\ell$ of granular zeolite available under the trademark of HSZ-320NAD, from Toso.

The concentration of methyl chloroformate was changed to 2400 ppm by volume.

The gas hourly space velocity (GHSV) of the mixed gas was changed to 20,000 $hr^{-1}$.

The resultant mixed gas, contained 430 ppm by volume of methyl chloroformate and 1970 ppm by volume of methyl chloride. Namely, methyl chloroformate was decomposed at a conversion of 82%.

Example 5

The same procedures as in Example 1 were carried out with the following exceptions.

The granular activated carbon was replaced by 10 m$\ell$ of granular zeolite (HSZ-320NAD, Toso).

The concentration of methyl chloroformate was changed to 2,000 ppm by volume.

The gas hourly space velocity (GHSV) of the mixed gas was changed to 3,000 $hr^{-1}$.

In the resultant mixed gas, no methyl chloroformate was detected, and the concentration of methyl chloride was 2,000 ppm by volume. It was confirmed that methyl chloroformate was entirely decomposed at a conversion of 100%.

Example 6

The same procedures as in Example 1 were carried out with the following exceptions.

The granular activated carbon was replaced by 5 m$\ell$ granular titania having a 24 mesh size and available under the trademark of DC-3144, from Diamond Catalyst Co.

The concentration of methyl chloroformate was changed to 2,000 ppm by volume.

The gas hourly space velocity (GHSV) of the mixed gas was changed to 6,000 $hr^{-1}$.

The resultant mixed gas contained 800 ppm by volume of methyl chloroformate, and 1,200 ppm by volume of methyl chloride. It was confirmed that methyl chloroformate was decomposed at a conversion of 60%.

## Example 7

The same procedures as in Example 1 were carried out with the following exceptions.

The granular activated carbon was replaced by 1 m$\ell$ of granular $\gamma$-alumina available under the trademark of KHA-24, from Sumitomo Kagaku Kogyo K.K.

The concentration of methyl chloroformate was changed to 1,250 ppm by volume.

The gas hourly space velocity (GHSV) of the mixed gas was changed to 20,000 hr$^{-1}$.

The resultant mixed gas contained 440 ppm by volume of methyl chloroformate and 810 ppm by volume of methyl chloride. It was confirmed that methyl chloroformate was decomposed at a conversion of 65%.

## Example 8

The same procedures as in Example 1 were carried out with the following exceptions.

The granular activated carbon was replaced by 5 m$\ell$ of granular activated carbon having a specific surface area of 1,300 m$^2$/g.

The concentration of methyl chloroformate was changed to 1,000 ppm by volume.

The gas hourly space velocity (GHSV) of the mixed gas was changed to 6,000 hr$^{-1}$. The decomposition temperature was changed to 40°C.

In the resultant mixed gas, no methyl chloroformate was detected, and the concentration of methyl chloride was 1,000 ppm by volume. It was confirmed that methyl chloroformate was entirely decomposed at a conversion of 100%.

## Example 9

The same procedures as in Example I were carried out with the following exceptions.

The granular activated carbon was replaced by 5 m$\ell$ of granular activated carbon having a specific surface area of 490 m$^2$/g.

The concentration of methyl chloroformate was changed to 1,000 ppm by volume.

The gas hourly space velocity (GHSV) of the mixed gas was changed to 6,000 hr$^{-1}$. The reaction temperature was changed to 20°C.

In the resultant mixed gas, no methyl chloroformate was detected, and the concentration of methyl chloride was 1,000 ppm by volume. It was confirmed that methyl chloroformate was entirely decomposed at a conversion of 100%.

## Example 10

The same procedures as in Example 1 were carried out with the following exceptions.

The granular activated carbon was replaced by 1 m$\ell$ of granular activated carbon having a specific surface area of 490 m$^2$/g.

The concentration of methyl chloroformate was changed to 2,000 ppm by volume.

The gas hourly space velocity (GHSV) of the mixed gas was changed to 30,000 hr$^{-1}$. The reaction temperature was changed to 40°C.

In the resultant mixed gas, no methyl chloroformate was detected, and the concentration of methyl chloride was 2,000 ppm by volume. It was confirmed that methyl chloroformate was entirely decomposed at a conversion of 100%.

## Example 11

The same procedures as in Example 1 were carried out with the following exceptions.

The granular activated carbon was replaced by 1 m$\ell$ of granular activated carbon having a specific surface area of 490 m$^2$/g.

The concentration of methyl chloroformate was changed to 2,000 ppm by volume.

The gas hourly space velocity (GHSV) of the mixed gas was changed to 30,000 hr$^{-1}$. The reaction temperature was changed to 20°C.

The resultant mixed gas contained 400 ppm by volume of methyl chloroformate and 1,600 ppm by volume of methyl chloride. It was confirmed that methyl chloroformate was decomposed at a conversion of 80%.

## Comparative Example 1

The same procedures as in Example 1 were carried out with the following exceptions.

The granular activated carbon was replaced by 10 m$\ell$ of glass beads available under the trademark of Pilex from Fuji Stone Co.

The concentration of methyl chloroformate was changed to 1,000 ppm by volume.

The gas hourly space velocity (GHSV) of the mixed gas was changed to 3,000 $hr^{-1}$.

The resultant mixed gas contained 1,000 ppm by volume of methyl chloroformate, and no methyl chloride was detected. It was confirmed that methyl chloroformate was not decomposed.

The reaction conditions and results of Examples 1 to 11 and Comparative Example 1 are shown in Table 1.

## Table 1

| Item | | | Decomposition catalyst | | Decomposition temperature | Concentration of methyl chloroformate | Gas hourly space velocity (GHSV) | Conversion (decomposition percentage) of methyl chloroformate |
|---|---|---|---|---|---|---|---|---|
| Example No. | | | Type | Amount (ml) | (°C) | (ppm by volume) | (hr⁻¹) | (%) |
| Example | | 1 | Activated carbon(*)₁ | 5 | 120 | 500 | 4000 | 100 |
| | | 2 | Activated alumina | 1 | 120 | 840 | 20000 | 100 |
| | | 3 | Activated alumina | 1 | 80 | 800 | 20000 | 65 |
| | | 4 | Zeolite | 5 | 120 | 2400 | 6000 | 82 |
| | | 5 | Zeolite | 10 | 120 | 2000 | 3000 | 100 |
| | | 6 | Titania | 5 | 150 | 2000 | 6000 | 60 |
| | | 7 | Alumina | 1 | 120 | 1250 | 20000 | 65 |
| | | 8 | Activated carbon(*)₂ | 5 | 40 | 1000 | 6000 | 100 |
| | | 9 | Activated carbon(*)₃ | 5 | 20 | 1000 | 6000 | 100 |
| | | 10 | Activated carbon(*)₃ | 1 | 40 | 2000 | 30000 | 100 |
| | | 11 | Activated carbon(*)₃ | 1 | 20 | 2000 | 30000 | 80 |
| Comparative Example | | 1 | Glass beads | 10 | 120 | 1000 | 3000 | 0 |

Note:   (*)₁ ... Trademark:  Shirasagi, made by Takeda
                  Yakuhin K.K.
        (*)₂ ... Trademark:  Kureha Beads, made by Kureha
                  Kagaku K.K.
        (*)₃ ... Trademark:  Molsiebon, made by Takeda
                  Yakuhin K.K.

As mentioned above, in accordance with the method of the present invention, a chloroformic acid ester, for example, alkyl chloroformate, can be easily and econominally decomposed and converted to a corresponding hydrocarbon chloride, for example, alkyl chloride at a high efficency. Even if the concentration of the chloroformic acid ester in a mixed gas is low, this chloroformic acid ester can be selectively decomposed and converted to the corresponding hydrocarbon chloride at a very high conversion, and easily removed from the mixed gas.

Example 12

A stainless steel reactor tube having an inside diameter of 27 mm and a length of 500 mm and equipped with an outside heating jacket was fixed vertically and a bottom portion of the tube was packed with 3.5 m$\ell$ of granular activated carbon (Shirasagi, Takeda Yakuhin) to form a decomposition reaction layer, and then on the decomposition reaction layer, 25 m$\ell$ of a solid catalyst consisting of granular activated carbon carrier and a catalytic component consisting of 1% by weight of palladium chloride and 1.2% by weight of copper (II) chloride each in terms of the metal, and carried on the carrier were placed to provide a carbonylation reaction layer.

By circulating a heating medium through the outside heating jacket, the inside temperature of the reactor tube was maintained at 100°C.

Then, a material gas containing 10% by volume of methyl nitrite, 20% by volume of carbon monoxide, 4% by volume of nitrogen monoxide, 8% by volume of methyl alcohol, 50 ppm by volume of hydrogen chloride and the balance consisting of nitrogen gas was fed into the reactor tube through a top inlet of the reactor tube at a flow rate of 100N liters/hr corresponding to a gas hourly space velocity (GHSV) of 28,600 hr$^{-1}$ (based on the decomposition reaction layer) under a reaction pressure of 2.94 bar (3 kg/cm$^2$G) for 10 hours, to effect a carbonylation and a decomposition. During the reaction procedures, the temperatures of the carbonylation reaction layer and the decomposition reaction layer were maintained at a level of 105°C to 115°C and 110°C, respectively.

The resultant reaction product mixture gas delivered from the reactor tube was subjected to a gas chromatographic analysis to determine the contents of methyl chloroformate and methyl chloride in the gas mixture.

As a result, no methyl chloroformate was detected, and the concentration of methyl chloride in the delivered gas mixture was 47 ppm by volume.

The carbonylation reaction product was collected by flowing the gas mixture through a ice-cooled methyl alcohol bath, and subjected to an ion chromatographic analysis. The collected product contained methyl chloroformate and hydrogen chloride, in a total amount of 1 ppm by volume. Also, it was confirmed that dimethyl carbonate was produced at a space time yield (STY) of 380 g/liter · hr and at a selectivity of 88% based on the molar amount of carbon monoxide.

In this example, and Examples 13 and 14 and Comparative Examples 2 and 3, the space time yield (STY) of the targeted carbonylation product such as dimethyl carbonate in g/liter · hr was determined in accordance with the equation (I):

$$STY \text{ (g/liter · hr)} = a/(b \times \theta) \tag{I}$$

wherein $\theta$ represents a catalytic reaction time in hours of carbon monoxide with a nitrous acid ester such as methyl nitrite, a represents an amount in grams of the resultant carbonic acid diester such as dimethyl carbonate produced during the catalytic reaction time, and b represents a volume in liters of the carbonylation reaction layer formed in the reactor tube.

Also, in each of Examples 12 to 14 and Comparative Examples 2 and 3, the selectivity in % of the targeted carbonylation product such as dimethyl carbonate was determined based on the molar amount of carbon monoxide supplied to the reaction and in accordance with the equation (II):

$$X(\%) = \{c/(c + 2 \times d + e)\} \times 100 \tag{II}$$

wherein c, d and e respectively represent molar amounts of the aimed carbonylation product such as dimethyl carbonate, a corresponding oxalic acid diester such as dimethyl oxalate, and carbon dioxide.

Example 13

The same procedures as in Example 12 were carried out with the following exceptions. In the formation of the decomposition reaction layer, 3.5 m$\ell$ of $\gamma$-alumina (available under the trademark of KHA-24, from Sumitomo Kagaku Kogyo K.K.) were packed in place of the granular activated carbon.

The gas mixture delivered from the reactor contained methyl chloroformate in a concentration of 4 ppm by volume and methyl chloride in a concentration of 43 ppm by volume.

After flowing through an ice-cooled methyl alcohol bath, the collected reaction product contained methyl chloroformate and hydrogen chloride in a total amount of 5 ppm by volume in terms of concentration in the gas.

It was confirmed that dimethyl carbonate was produced in a space time yield (STY) of 375 g/liter · hr and at a selectivity of 90% based on the amount of carbon monoxide used.

Comparative Example 2

The same procedures as in Example 12 were carried out with the following exceptions.

No decomposition reaction layer was provided.

The delivered gas from the reactor contained methyl chloroformate in a concentration of 18 ppm by volume, and methyl chloride in a concentration of 27 ppm by volume.

After flowing the delivered gas through an ice-cooled methyl alcohol bath, the collected reaction product contained methyl chloroformate and hydrogen chloride in a total amount of 25 ppm by volume in terms of concentration in the gas.

The dimethyl carbonate was produced in a space time yield (STY) of 377 g/liter · hr and at a selectivity of 88% based on the amount of carbon monoxide used.

Example 14

The same procedures as in Example 12 were carried out with the following exceptions.

In the formation of the decomposition reaction layer, 5 m$\ell$ of $\gamma$-alumina grains (KHA-24) were packed in place of the granular activated carbon, and the carbonylation reaction layer was formed from 25 m$\ell$ of a catalyst which consisted of a carrier consisting of $\gamma$-alumina grains (KHA-24) and a catalytic component consisting of 1.5% by weight of a lithium chloropalladate in terms of palladium, in accordance with EP-A-0523508 (DE-B-412 3603).

The concentrations of methyl nitrite and hydrogen chloride were changed to 18% by volume and 1,000 ppm by volume, respectively.

The temperatures of the carbonylation reaction layer and the decomposition reaction layer were 108°C to 120°C and 114°C, respectively.

After the completion of the reactions, the reaction product was analysed. The gas delivered from the reactor contained 15 ppm by volume of methyl chloroformate and 970 ppm by volume of methyl chloride.

After passing the delivered gas through an ice-cooled methyl alcohol bath, the collected product contained methyl chloroformate and hydrogen chloride in a total amount of 20 ppm by volume in terms of concentration in the gas.

The resultant dimethyl carbonate was obtained in a space time yield of 570 g/liter · hr and at a selectivity of 88% based on the amount of carbon monoxide employed.

Comparative Example 3

The same procedures as in Example 14 were carried out with the following exceptions.

No decomposing reaction layer was formed.

The gas delivered from the reactor contained 537 ppm by volume of methyl chloroformate and 455 ppm of methyl chloride.

After passing the delivered gas through an ice-cooled methyl alcohol bath, the collected product contained methyl chloroformate and hydrogen chloride in a total amount of 550 ppm by volume in terms of concentration in the gas.

The resultant dimethyl carbonate had a space time yield (STY) of 565 g/liter · hr and a selectivity of 88% based on the amount of carbon monoxide employed.

Example 15

The same procedures as in Example 12 were carried out with the exceptions as indicated below.

In the formation of the decomposition reaction layer in the reaction tube, 3 m$\ell$ of granular $\gamma$-alumina (KHA-24) were used in place of the granular activated carbon.

The carbonylation reaction layer was formed from 15 m$\ell$ of a catalyst for the production of dimethyl succinate. The catalyst consisted of a carrier consisting of the granular $\gamma$-alumina (KHA-24) and a catalytic component consisting of 1% by weight of palladium chloride and 1.2% by weight of copper (II) chloride and carried on the carrier.

A material gas consisting of 5% by volume of methyl nitrite, 20% by volume of carbon monoxide, 15% by volume of ethylene, 4% by volume of nitrogen monoxide, 8% by volume of methyl alcohol, 800 ppm by volume of hydrogen chloride and the balance consisting of a nitrogen gas was fed into the reactor at a flow rate of 50 N · liter/hr corresponding to a gas hourly space velocity (GHSV) of 16,700 hr$^{-1}$ based on the decomposition reaction layer. The temperatures of the carbonylation reaction layer and the decomposition reaction layer were 105°C to 115°C and 108°C, respectively.

The gas delivered from the reactor was subjected to a gas chromatographic analysis. It was found that the delivered gas contained 10 ppm by volume of methyl chloroformate and 770 ppm by volume of methyl chloride.

After passing the delivered gas through an ice-cooled methyl alcohol gas, the collected reaction product was subjected to an ion chromatographic analysis. It was found that the collected reaction product contained methyl chloroformate and hydrogen chloride in a total amount of 15 ppm by volume in terms of concentration in the gas.

The resultant dimethyl succinate was produced in a space time yield (STY) of 230 g/liter·hr, at a selectivity of 93% based on the amount of carbon monoxide employed.

The space time yield (STY, g/liter·hr) of dimethyl succinate was calculated in accordance with the equation (III):

$$STY \text{ (g/liter} \cdot \text{hr)} = f/(b \times \theta') \qquad \text{(III)}$$

wherein $\theta'$ represents a catalytic reaction time in hours of carbon monoxide with ethylene and methyl nitrite, f represents an amount in grams of dimethyl succinate produced during the catalytic reaction time and b represents the volume in liters of the carbonylation reaction layer.

Also the conversion Y of the dimethyl succinate based on the amount of carbon monoxide employed was determined in accordance with the equation (IV):

$$Y(\%) = \{2 \times g/(2 \times g + 2 \times h + i + j)\} \times 100 \qquad \text{(IV)}$$

wherein g, h, i and j respectively represent molar amounts of the resultant dimethyl succinate, dimethyl oxalate, dimethyl carbonate and carbon dioxide produced during the catalytic reaction time $\theta'$ in hours.

The results of Examples 12 to 15 and Comparative Examples 2 and 3 are shown in Table 2.

In the Table 2, the concentration $C_1$ in ppm of hydrogen chloride in the ice-cooled, collected reaction product was calculated in accordance with the equation (V):

$$C_1 = C_2 - C_3 \qquad \text{(V)}$$

wherein $C_2$ represents a total concentration in ppm of the chlorine compounds contained in the ice-cooled, collected reaction product and $C_3$ represents a concentration in ppm of methyl chloroformate contained in the gas delivered from the reactor.

Table 2 clearly shows that when a carbonylation reaction of carbon monoxide with a nitrous acid ester in the presence of a solid catalyst and in the presence of a chlorine-containing substance (for example, hydrogen chloride, chlorine and/or a chloroformic acid ester) is carried out in the gas phase, and the resultant reaction product gas mixture

Table 2

| Item | | HCl content in material gas for carbonylation reaction (ppm) | Decomposition catalyst | | Decomposition temperature (°C) | Methyl chloroformate content (C₃) in gas delivered from reactor (ppm) | CH₃Cl content in gas delivered from reactor (ppm) | Chlorine compound content (C₂) in ice-cooled, collected reaction product (ppm) | HCl content (C₁) in ice-cooled, collected reaction product (ppm) | Aimed ester compound (dimethyl carbonate or succinate) | |
|---|---|---|---|---|---|---|---|---|---|---|---|
| | | | Type | Amount (ml) | | | | | | Space time yield (STY) (g/ℓ·hr) | Selectivity (%) |
| Example | 12 | 50 | Activated carbon | 3.5 | 110 | 0 | 47 | 1 | 1 | 380 | 88 |
| | 13 | 50 | γ-alumina | 3.5 | 110 | 4 | 43 | 5 | 1 | 375 | 90 |
| Comparative Example 2 | | 50 | ―――― | | 110 | 18 | 27 | 25 | 7 | 377 | 88 |
| Example 14 | | 1000 | γ-alumina | 5.0 | 114 | 15 | 970 | 20 | 5 | 570 | 88 |
| Comparative Example 3 | | 1000 | ―――― | | 114 | 537 | 455 | 550 | 13 | 565 | 88 |
| Example 15 | | 800 | γ-alumina | 3.0 | 108 | 10 | 770 | 15 | 5 | 230 | 93 |

Note:   $C_1 = C_2 - C_3$

13

containing, as a by-product or additive, a small amount of a chloroformic acid alkyl ester in addition to the targeted ester compound (for example, dimethyl carbonate or succinate) is subjected to the catalytic decomposition step of the present invention, the chloroformic acid alkyl ester can be decomposed and converted to the corresponding alkyl chloride, for example, methyl chloride at a high conversion. Accordingly, the resultant product mixture can be refined without corroding the refining apparatus.

## Claims

1. A method of decomposing a chloroformic acid alkyl ester, comprising the step of:

   bringing a gas containing a chloroformic acid alkyl ester into contact with a decomposition catalyst comprising at least one member selected from the group consisting of activated carbon and inorganic oxides, to convert the chloroformic acid alkyl ester to a corresponding alkyl chloride.

2. The method as claimed in claim 1, wherein the chloroformic acid alkyl ester is an alkyl chloroformate, of which the alkyl group has 1 to 4 carbon atoms.

3. The method as claimed in claim 1, wherein the chloroformic acid alkyl ester-containing gas contains the chloroformic acid alkyl ester in an amount of from 10 ppm to 100% by volume.

4. The method as claimed in claim 1, wherein the activated carbon has a specific surface area of 10 $m^2$/g or more.

5. The method as claimed in claim 1, wherein the inorganic oxides are selected from the group consisting of alumina, silica-alumina, synthetic zeolites, natural zeolites, silica, titania and zirconia.

6. The method as claimed in claim 1, wherein the inorganic oxides have a specific surface area of 10 $m^2$/g or more.

7. The method as claimed in claim 1, wherein the inorganic oxide is γ-alumina having a specific surface area of 10 $cm^2$/g or more.

8. The method as claimed in claim 1, wherein the decomposition of the chloroformic acid alkyl ester is carried out at a temperature of from 0°C to 220°C.

9. The method as claimed in claim 1, wherein the chloroformic acid alkyl ester-containing gas is a reaction product mixture derived from a gas phase catalytic carbonylation reaction of carbon monoxide with a nitrous acid ester in the presence of a solid catalyst and in the presence of a small amount of a chlorine-containing substance, and the resultant alkyl chloride is removed from the reaction product mixture.

10. The method as claimed in claim 9, wherein the gas phase catalytic carbonylation reaction is a carbonic acid ester-synthesis reaction, a dicarboxylic acid diester-synthesis reaction or a succinic acid diester-synthesis reaction.

11. The method as claimed in claim 9, wherein the chlorine-containing substance comprises at least one member selected from the group consisting of chlorine, hydrogen chloride and chloroformic acid alkyl esters.

12. The method as claimed in claim 9, wherein the chloroformic acid alkyl ester-containing gas is a reaction product mixture derived from a gas phase carbonic acid ester-synthesis reaction and the chlorine-containing substance is hydrogen chloride.

## Patentansprüche

1. Verfahren zum Zersetzen eines Chlorameisensäure-Alkylesters, umfassend die Schritte von:

   Inkontaktbringen eines Gases, welches einen Chlorameisensäure-Alkylester enthält, mit einem Zersetzungskatalysator, welcher mindestens ein Mitglied, ausgewählt aus der aus Aktivkohle und anorganischen Oxiden bestehenden Gruppe umfaßt, um den Chlorameisensäure-Alkylester in ein korrespondierendes Alkylchlorid umzuwandeln.

2. Verfahren nach Anspruch 1, worin der Chlorameisensäure-Alkylester ein Chlorameisensäure-Alkylester ist, bei welchem die Alkylgruppe 1 - 4 Kohlenstoffatome aufweist.

3. Verfahren nach Anspruch 1, worin das Chlorameisensäure-Alkylester enthaltende Gas den Chlorameisensäurealkylester in einer Menge von 10 ppm - 100 Vol.% enthält.

4. Verfahren nach Anspruch 1, worin die Aktivkohle einen spezifischen Oberflächenbereich von 10 m$^2$/g oder mehr aufweist.

5. Verfahren nach Anspruch 1, worin die anorganischen Oxide ausgewählt sind aus der aus Aluminiumoxid, silikatischem Aluminiumoxid, synthetischen Zeolithen, natürlichen Zeolithen, Siliziumdioxid, Titandioxid und Zirkondioxid bestehenden Gruppe.

6. Verfahren nach Anspruch 1, worin die anorganischen Oxide einen spezifischen Oberflächenbereich von 10 m$^2$/g oder mehr aufweisen.

7. Verfahren nach Anspruch 1, worin das anorganische Oxid $\gamma$-Aluminiumoxid mit einem spezifischen Oberflächenbereich von 10 cm$^2$/g oder mehr ist.

8. Verfahren nach Anspruch 1, worin die Zersetzung des Chlorameisensäure-Alkylesters bei einer Temperatur von 0°C bis 220°C durchgeführt wird.

9. Verfahren nach Anspruch 1, worin das Chlorameisensäure-Alkylester enthaltende Gas eine Reaktionsproduktmischung ist, welche aus einer katalytischen Gasphasen-Carbonylierungsreaktion von Kohlenmonoxid mit einem Ester der salpetrigen Säure in Gegenwart eines festen Katalysators und in Gegenwart einer geringen Menge einer chlorhaltigen Verbindung gewonnen wird, und wobei das resultierende Alkylchlorid aus der Reaktionsproduktmischung entfernt wird.

10. Verfahren nach Anspruch 9, worin die katalytische Gasphasencarbonylierungsreaktion eine Synthesereaktion für Carbonsäureester, eine Synthesereaktion für Dicarbonsäurediester oder eine Synthesereaktion für Bernsteinsäurediester ist.

11. Verfahren nach Anspruch 9, worin die chlorhaltige Verbindung mindestens ein Mitglied enthält, ausgewählt aus der aus Chlor, Chlorwasserstoff und Chlorameisensäurealkylester bestehenden Gruppe.

12. Verfahren nach Anspruch 9, worin das Chlorameisensäure-Alkylester enthaltende Gas eine Reaktionsproduktmischung ist, welche aus der Gasphasensynthesereaktion für Carbonsäureester erhalten wird, und die Chlor enthaltende Verbindung Chlorwasserstoff ist.

**Revendications**

1. Procédé de décomposition d'un ester alkylique d'acide chloroformique, comprenant l'étape consistant :

   à mettre un gaz contenant un ester alkylique d'acide chloroformique en contact avec un catalyseur de décomposition comprenant au moins un composant choisi parmi le carbone activé et les oxydes inorganiques, afin de convertir l'ester alkylique d'acide chloroformique en un chlorure d'alkyle correspondant.

2. Procédé selon la revendication 1, dans lequel l'ester alkylique d'acide chloroformique est un chloroformiate d'alkyle dont le groupe alkyle comporte de 1 à 4 atomes de carbone.

3. Procédé selon la revendication 1, dans lequel le gaz contenant l'ester alkylique d'acide chloroformique, contient l'ester alkylique d'acide chloroformique selon une quantité de 10 ppm à 100 % en volume.

4. Procédé selon la revendication 1, dans lequel le carbone activé a une surface spécifique de 10 m$^2$/g ou plus.

5. Procédé selon la revendication 1, dans lequel les oxydes inorganiques sont choisis parmi l'alumine, la silice-alumine, les zéolites synthétiques, les zéolites naturelles, la silice, l'oxyde de titane et l'oxyde de zirconium.

6. Procédé selon la revendication 1, dans lequel les oxydes inorganiques ont une surface spécifique de 10 m$^2$/g ou plus.

7. Procédé selon la revendication 1, dans lequel l'oxyde inorganique est l'alumine $\gamma$ ayant une surface spécifique de

$10 \ cm^2/g$ ou plus.

8. Procédé selon la revendication 1, dans lequel la décomposition de l'ester alkylique d'acide chloroformique est effectuée à une température de 0 °C à 220 °C.

9. Procédé selon la revendication 1, dans lequel le gaz contenant l'ester alkylique d'acide chloroformique est un mélange de produits de réaction dérivé d'une réaction de carbonylation catalytique en phase gazeuse du monoxyde de carbone avec un ester d'acide nitreux, en présence d'un catalyseur solide et d'une faible quantité d'une substance chlorée, et le chlorure d'alkyle résultant est séparé du mélange de produits de réaction.

10. Procédé selon la revendication 9, dans lequel la réaction de carbonylation catalytique en phase gazeuse est une réaction de synthèse d'ester d'acide carbonique, une réaction de synthèse de diester d'acide dicarboxylique ou une réaction de synthèse de diester d'acide succinique.

11. Procédé selon la revendication 9, dans lequel la substance chlorée comprend au moins un composant choisi parmi le chlore, le chlorure d'hydrogène et les esters alkyliques d'acide chloroformique.

12. Procédé selon la revendication 9, dans lequel le gaz contenant l'ester alkylique d'acide chloroformique, est un mélange de produits de réaction dérivé d'une réaction de synthèse d'ester d'acide carbonique en phase gazeuse, et la substance chlorée est le chlorure d'hydrogène.